# EUROPEAN PATENT SPECIFICATION

(11) **EP 3 553 046 B1**
(45) Date of publication and mention of the grant of the patent: **10.03.2021**
(21) Application number: 17878160.5
(22) Date of filing: 08.12.2017
(51) Int. Cl.: C07C 231/12, C07C 233/18, C07B 61/00

(54) **PRODUCTION METHOD FOR N ( HYDROXYETHYL) FORMAMIDE, PRODUCTION METHOD FOR N ( ALKOXYETHYL) FORMAMIDE, PRODUCTION DEVICE FOR N ( HYDROXYETHYL) FORMAMIDE, AND PRODUCTION DEVICE FOR N ( ALKOXYETHYL) FORMAMIDE**
HERSTELLUNGSVERFAHREN FÜR N(HYDROXYETHYL)FORMAMID, HERSTELLUNGSVERFAHREN FÜR N(ALKOXYETHYL)FORMAMID, HERSTELLUNGSVORRICHTUNG FÜR N(HYDROXYETHYL)FORMAMID UND HERSTELLUNGSVORRICHTUNG FÜR N(ALKOXYETHYL)FORMAMID
PROCÉDÉ DE PRODUCTION DE N-( -HYDROXYÉTHYL) FORMAMIDE, PROCÉDÉ DE PRODUCTION DE N-( -ALKOXYÉTHYL) FORMAMIDE, DISPOSITIF DE PRODUCTION DE N-( -HYDROXYÉTHYL) FORMAMIDE, ET DISPOSITIF DE PRODUCTION DE N-( -ALKOXYÉTHYL) FORMAMIDE

(30) Priority: 08.12.2016 JP 2016238206
(43) Date of publication of application: 16.10.2019
(73) Proprietor: Mitsubishi Chemical Corporation, Tokyo 100-8251 (JP)
(72) Inventor: TERAMOTO Kouji, Tokyo 100-8251 (JP); NISHIMURA Hitoshi, Tokyo 100-8251 (JP)
(74) Representative: Hoffmann Eitle
(86) International application number: PCT/JP2017/044152
(87) International publication number: WO 2018/105720

(56) References cited:
- WO-A1-2018/135574
- JP-A- H05 319 481
- JP-A- H06 298 713
- US-A- 5 574 185
- "Chemical equipment editorial department section Separate volume of ''chemcial equipment'' Basicknowledge on powder technology", KOGYO CHOSAKAI PUBLISHING CO., 9 September 1997 (1997-09-09), pages 78-82 , 136-141,
- "Storage and supply of supplementary granular particles", KAGAKU KOGYOSHA KABUSHIKI KAISAHA, 1 March 1983 (1983-03-01), pages 127-195,
- "Separate volume of chemical equipment-Acqusistion of granular particels technology for procurement plan and model selection", KOGYO CHOSAKAI PUBLISING CO., 15 September 1976 (1976-09-15), pages 40-52 , 135-153,
- ITO, MITSUHIRO: "Passage : NDL Illustration Basic knowledge of "Powder equipment", Basic knowledge of "Powder Equipment / Equipment", 10 September 2001 (2001-09-10), pages 60-79, XP009518017,

## Description

### TECHNICAL FIELD

The present invention relates to a method for producing N-(α-hydroxyethyl)formamide, a method for producing N-(α-alkoxyethyl)formamide, a device for producing N-(α-hydroxyethyl)formamide, and a device for producing N-(α-alkoxyethyl)formamide.

### BACKGROUND ART

N-(α-hydroxyethyl)formamide and N-(α-alkoxyethyl)formamide are important substances as intermediate raw materials for N-vinylformamide.

N-(α-hydroxyethyl)formamide is obtained by reacting (hydroxylation reaction) formamide with acetaldehyde in the presence of a basic catalyst. N-(α-alkoxyethyl)formamide is obtained by reacting (alkoxylation reaction) N-(α-hydroxyethyl)formamide with an alcohol in the presence of an acid catalyst (see Patent Document 1).

In the hydroxylation reaction, the basic catalyst is supplied to the reaction system, for example, by the following method.

First, the basic catalyst stored in the catalyst storage tank first passes through the powder feeder and the amount thereof supplied to the mixing tank is adjusted. The basic catalyst of which the amount supplied has been adjusted is supplied to the mixing tank through the catalyst supply pipe and mixed with formamide in the mixing tank. The basic catalyst is supplied to the reaction system as this basic catalyst mixed with formamide is supplied to the hydroxylation reaction tank through the catalyst-mixed liquid supply pipe. The formamide supplied to the reaction system reacts with acetaldehyde in the hydroxylation reaction tank to form a slurry-like reaction product.

### CITATION LIST

### PATENT DOCUMENT

Patent Document 1: JP 6-298713 A

US 5,574,185 discloses a method for producing N-(α-alkoxyethyl)formamide comprising reacting formamide with acetaldehyde in the presence of a basic catalyst which is potassium bicarbonate or potassium hydrogen phosphate, thereby producing N-(α-hydroxyethyl)formamide, and reacting the N-(α-hydroxyethyl) formamide with a primary or secondary alcohol in the presence of an acid catalyst, thereby giving N-(α-alkoxyethyl)formamide.

### SUMMARY OF THE INVENTION

### PROBLEM TO BE SOLVED BY THE INVENTION

However, in the conventional method for producing N-(α-hydroxyethyl)formamide, there is a case in which a part of the basic catalyst is retained at the bottom portion of the catalyst storage tank, in the powder feeder, and in the catalyst supply pipe and the amount of the basic catalyst supplied is not sufficiently quantitative when the basic catalyst is supplied from the catalyst storage tank to the mixing tank even if the basic catalyst is accurately measured by the amount required and stored in the catalyst storage tank.

In addition, there is a case in which the solid components in the slurry accumulate in the valve which controls the amount of the hydroxylation reaction slurry supplied and the flow rate of the hydroxylation reaction slurry decreases when the hydroxylation reaction slurry is supplied to the alkoxylation reaction tank.

In addition, in the conventional method for producing N-(α-hydroxyethyl)formamide, the reaction yield of the reaction (hydroxylation reaction) for obtaining N-(α-hydroxyethyl)formamide from formamide decreases when a sufficient amount of basic catalyst cannot be supplied to the reaction system.

In addition, according to the conventional method for producing N-(α-alkoxyethyl)formamide, the reaction yield of the reaction (alkoxylation reaction) for obtaining N-(α-alkoxyethyl)formamide from N-(α-hydroxyethyl)formamide decreases, and as a result, the purity and quality of N-vinylformamide decrease.

The invention has been made in view of the above circumstances, and an object thereof is to provide a method for producing N-(α-hydroxyethyl)formamide by which the amount of the basic catalyst retained at the bottom portion of the catalyst storage tank, in the powder feeder, and in the catalyst supply pipe decreases, the amount of the basic catalyst to be supplied from the catalyst storage tank to the mixing tank becomes quantitative, the flow rate of the hydroxylation reaction slurry does not decrease, and the reaction yield of hydroxylation reaction is improved, a method for producing N-(α-alkoxyethyl)formamide by which the reaction yield of alkoxylation is improved and N-(α-alkoxyethyl)formamide exhibiting excellent quality is obtained, a device for producing N-(α-hydroxyethyl)formamide, and a device for producing N-(α-alkoxyethyl)formamide.

### MEANS FOR SOLVING PROBLEM

The invention has the configuration as defined in the claims.

### EFFECT OF THE INVENTION

According to the invention, it is possible to provide a method for producing N-(α-hydroxyethyl)formamide by which the amount of the basic catalyst retained at the bottom portion of the catalyst storage tank, in the powder feeder, and in the catalyst supply pipe decreases, the amount of the basic catalyst to be supplied from the catalyst storage tank to the mixing tank becomes quantitative, the flow rate of the hydroxylation reaction slurry does not decrease, and the reaction yield of hydroxylation reaction is improved, a method for producing N-(α-alkoxyethyl)formamide by which the reaction yield of alkoxylation is improved and N-(α-alkoxyethyl)formamide exhibiting excellent quality is obtained, a device for producing N-(α-hydroxyethyl)formamide, and a device for producing N-(α-alkoxyethyl)formamide.

### BRIEF DESCRIPTION OF DRAWINGS

Fig. 1 is a schematic configuration diagram illustrating a device for producing N-(α-hydroxyethyl)formamide; and
Fig. 2 is a schematic configuration diagram illustrating a device for producing N-(α-alkoxyethyl)formamide used in Examples and Comparative Examples.

### MODE(S) FOR CARRYING OUT THE INVENTION

### (Method for producing N-(α-hydroxyethyl)formamide)

The method for producing N-(α-hydroxyethyl)formamide of the invention includes the following step (1) and step (2).

Hereinafter, the respective steps will be described in detail.

### [Step (1)]

The step (1) is a step of mixing a basic catalyst with formamide and obtaining a mixture (reaction raw material liquid A) of a basic catalyst and formamide.

In the invention, the basic catalyst is supplied to the mixing tank while vibrating at least a part of the catalyst supply facility.

The catalyst supply facility includes a catalyst storage tank for storing a basic catalyst, a powder feeder for supplying the basic catalyst, and a catalyst supply pipe for supplying the basic catalyst.

The basic catalyst stored in the catalyst storage tank first passes through the powder feeder so that the amount thereof supplied to the mixing tank is adjusted. The basic catalyst of which the amount supplied has been adjusted is then supplied to the mixing tank through the catalyst supply pipe and mixed with formamide in the mixing tank.

In the invention, it is preferable that the basic catalyst is filled in the powder feeder and then supplied to the mixing tank. It is preferable that the basic catalyst is filled in the powder feeder since it is possible to accurately measure the amount of the basic catalyst stored in the catalyst storage tank and to supply a regulated amount of the basic catalyst to the reaction system.

The means for generating vibration to at least a part of the catalyst supply facility is not particularly limited, but hammering (hammering) is preferable since it is possible to easily and inexpensively generate vibration. Specific examples of hammering may include an air pulse generator, an air knocker, an electric knocker, and vibration generators such as an ultrasonic vibrator, a piezoelectric vibrator, and a ball vibrator as well as a hammer, but the examples are not limited thereto. Among these, an air pulse generator, an air knocker, an electric knocker, and a vibration generator which impart vibration having a constant vibration frequency, can uniformly supply a constant amount of catalyst, and do not hinder the measuring of catalyst are preferable.

The site at which vibration is imparted to the catalyst supply facility by hammering may be the top portion of the catalyst storage tank or the side portion thereof, but the bottom portion of the catalyst storage tank is preferable. In addition, vibration may be imparted to the powder feeder and the catalyst supply pipe.

In the invention, the amount of the basic catalyst supplied from the catalyst supply facility to the mixing tank is preferably 90% by mass or more and more preferably 98% by mass or more of the amount of the basic catalyst stored in the catalyst supply facility.

When the amount of the basic catalyst supplied is less than 90% by mass of the amount of the basic catalyst stored in the catalyst supply facility, a decrease in the reaction efficiency of the hydroxylation reaction occurs and the amount of residual formamide and the amount of residual acetaldehyde increase in the next step (2). When N-vinylformamide is produced using N-(α-alkoxyethyl)formamide containing formamide and acetaldehyde as a raw material, a decrease in the purity, delay of polymerization, a decrease in the molecular weight after polymerization, and the like are caused. In addition, it is industrially disadvantageous to use a solvent in the hydroxylation reaction from the viewpoint of production efficiency since the filtration property deteriorates when separating the N-(α-hydroxyethyl)formamide precipitated and the solvent from each other in the step (3) in the (method for producing N-(α-alkoxyethyl)formamide) to be described later and the reaction yield of N-(α-hydroxyethyl)formamide decreases.

The specific aspect of mixing of the basic catalyst with formamide is not particularly limited, but it is preferable to spread liquid formamide in the mixing tank and to drop the basic catalyst thereon for mixing. In addition, it is preferable that the basic catalyst contained in the reaction raw material liquid A is present by being dissolved in formamide, but it is not particularly limited as long as the basic catalyst is present in the liquid of formamide as a mixture.

The basic catalyst is not particularly limited as long as it is a general basic compound, but it is preferably a weak basic salt composed of a strong base and a weak acid having a pKa value of from 4 to 15, and specific examples thereof may include sodium carbonate, sodium hydrogencarbonate, potassium carbonate, potassium hydrogencarbonate, lithium carbonate, lithium hydrogencarbonate, potassium phosphate, potassium monohydrogen phosphate, and sodium pyrophosphate. Among these, potassium hydrogencarbonate is preferable from the viewpoint of being able to decrease by-products (for example, aldol condensate of acetaldehyde) to be formed by the reaction of formamide with acetaldehyde in the next step (2).

The optimum value of the concentration of the basic catalyst varies depending on the impurities in formamide, particularly the acidic components and the like, but it is preferably from 0.3 to 3 moles, more preferably from 0.5 to 1.2 moles, still more preferably from 0.7 to 1 mole, and most preferably from 0.8 to 0.9 mole per 1 kg of formamide.

When the amount of the basic catalyst is more than the above range, the aldehydes are condensed with each other, crotonaldehyde or a more condensed substance is thus formed, a decrease in the reaction yield of N-(α-alkoxyethyl)formamide occurs, and also the polymerization of N-vinylformamide is delayed and a decrease in the molecular weight after polymerization is caused when these condensates are mixed into the raw materials for N-vinylformamide. In addition, when the amount of the basic catalyst supplied is set to be more than 3% by moles with respect to formamide, the solid components in the reaction slurry accumulate in the supply control valve for the hydroxylation reaction slurry and the flow rate of the reaction slurry decreases when the hydroxylation reaction slurry is supplied to the alkoxylation reaction tank (fourth tank) in the step (3) in the (method for producing N-(α-alkoxyethyl)formamide) to be described later. Furthermore, the filtration property deteriorates and it takes a long time to complete solid-liquid separation when separating the product which is solid and the reaction liquid which is liquid from each other in the step (3). On the other hand, when the amount of the basic catalyst is less than the above range, a decrease in the yield and inhibition of polymerization by the residual aldehyde as described above are caused.

Hence, it is preferable that the amount of the basic catalyst supplied is within the above range, and it is thus important to measure the amount thereof required and to supply the entire catalyst measured to the reaction tank.

### [Step (2)]

The step (2) is a step of bringing the reaction raw material liquid A obtained in the step (1) into contact with acetaldehyde and obtaining N-(α-hydroxyethyl)formamide.

The hydroxylation reaction proceeds as the reaction raw material liquid A and acetaldehyde are brought into contact with each other.

The specific aspect of the hydroxylation reaction is not particularly limited, but it is preferable to spread a mixed liquid (reaction raw material liquid B) of acetaldehyde and a solvent and to drop the reaction raw material liquid A thereon for the hydroxylation reaction.

In addition, a method is generally used in which crystals of N-(α-hydroxyethyl)formamide which is a product are precipitated after the reaction or at the middle stage of the reaction. In order to smoothly conduct this crystallization, an operation of adding a small amount of N-(α-hydroxyethyl)formamide crystals as a seed crystal in the middle of the dropwise addition of formamide mixed with the basic catalyst may be conducted.

As the solvent to be used in the hydroxylation reaction, for example, aliphatic hydrocarbons such as hexane, heptane, and cyclohexane; aromatic hydrocarbons such as benzene, toluene, and xylene; and halogenated hydrocarbons such as methylene chloride and chloroform are preferable from the viewpoint of crystallizing N-(α-hydroxyethyl)formamide.

The amount of the solvent used is preferably from 0.2 to 10 times the mass of formamide.

The molar ratio of formamide to acetaldehyde (formamide : acetaldehyde) is preferably from 1 : 1 to 1 : 10 and more preferably from 1 : 1 to 1 : 5. It is possible to increase the percent conversion of formamide by setting the molar ratio of acetaldehyde to be excessive.

The reaction temperature in the hydroxylation reaction can be measured by using a thermometer to be usually industrially used such as a thermocouple thermometer. The reaction temperature in the hydroxylation reaction is not particularly limited, and it is usually preferably about from -10°C to 100°C. The reaction temperature is preferably from 0°C to 40°C from the viewpoint of the hydroxylation reaction yield from formamide to N-(α-hydroxyethyl)formamide and of crystallizing the N-(α-hydroxyethyl)formamide formed. It is possible to increase the hydroxylation reaction yield by crystallizing N-(α-hydroxyethyl)formamide.

By precipitating the crystals of N-(α-hydroxyethyl)formamide, N-(α-hydroxyethyl)formamide can be subjected to the alkoxylation reaction in the step (3) in the (method for producing N-(α-alkoxyethyl)formamide) to be described later by simply collecting the crystals by a method such as filtration and further most of the solvent can be separated and recovered. In addition, it is also possible to adopt a method in which the solvent is not separated and recovered from the reaction mixture at this stage but the reaction mixture is subjected to the next alkoxylation reaction as it is.

The reaction mixture contains unreacted formamide and acetaldehyde, an aldol condensate of acetaldehyde which is a by-product of the hydroxylation reaction, the basic catalyst which is a catalyst, the reaction solvent, and the like in addition to N-(α-hydroxyethyl)formamide which is a reaction product of formamide with acetaldehyde.

### <Effect>

As described above, according to the method for producing N-(α-hydroxyethyl)formamide of the invention, the basic catalyst is supplied to the mixing tank while vibrating the catalyst storage tank in the step (1) and thus the amount of the basic catalyst retained at the bottom portion of the catalyst storage tank, in the powder feeder, and in the catalyst supply pipe decreases, a regulated amount of basic catalyst can be quantitatively supplied to the reaction system, the solid components do not accumulate in a valve which controls the amount of the reaction slurry supplied, and the flow rate of the hydroxylation reaction slurry does not decrease. The reaction yield of N-(α-alkoxyethyl)formamide increases by quantitatively supplying a regulated amount of catalyst.

### (Method for producing N-(α-alkoxyethyl)formamide)

The method for producing N-(α-alkoxyethyl)formamide of the invention includes a step (3).

Hereinafter, each step will be described in detail.

### [Step (3)]

The step (3) is a step of bringing N-(α-hydroxyethyl)formamide obtained by the (method for producing N-(α-hydroxyethyl)formamide) described above into contact with an alcohol in the presence of an acid catalyst and obtaining N-(α-alkoxyethyl)formamide.

The alkoxylation reaction proceeds as N-(α-hydroxyethyl)formamide and an alcohol are brought into contact with each other in the presence of an acid catalyst.

In the alkoxylation reaction, the reaction mixture obtained by the hydroxylation reaction described above may be used or N-(α-hydroxyethyl)formamide may be isolated from the reaction mixture and used.

The specific aspect of the alkoxylation reaction is not particularly limited, but for example, the alkoxylation reaction is easily accomplished by adding an acid catalyst to a mixture N-(α-hydroxyethyl)formamide and an alcohol or bringing these into contact with each other. In addition, a method in which an acid catalyst is dissolved in an alcohol in advance to prepare a catalyst solution and the catalyst solution is then added to N-(α-hydroxyethyl)formamide may be used.

The reaction temperature is preferably from -10°C to 60°C, more preferably from 0°C to 40°C, and still more preferably from 5°C to 30°C from the viewpoint of the reactivity of the alkoxylation reaction and the stability of N-(α-hydroxyethyl)formamide.

As the alcohol to be used in the alkoxylation reaction, a primary or secondary alcohol is used. Alcohols having from 1 to 8 carbon atoms are preferable and alcohols having from 1 to 4 carbon atoms are more preferable from the viewpoint of reactivity and handling property of N-(α-hydroxyethyl)formamide. Specific examples of the alcohol may include methanol, ethanol, n-propanol, n-butanol, isobutyl alcohol, n-pentanol, n-hexanol, n-heptanol, n-octanol, benzyl alcohol, 2-methoxyethanol, 2-ethoxyethanol, 2-propoxyethanol, 2-butoxyethanol, diethylene glycol monomethyl ether, ethylene glycol, propylene glycol, 1,4-butanediol, and diethylene glycol. Among these, a primary alcohol is preferable and methanol which has a low boiling point as a raw material and provides a product having a low boiling point is particularly preferable.

In order to increase the yield of product, it is preferable to use an excessive amount of alcohol, and specifically, a molar amount to be from 1.1 to 50 times the amount of N-(α-alkoxyethyl)formamide is preferable and a molar amount to be from 2.0 to 30 times the amount of N-(α-alkoxyethyl)formamide is more preferable.

Examples of the acid catalyst to be used in the alkoxylation reaction may include a mineral acid, an organic acid, an ion exchange resin exhibiting weak acidity or strong acidity, and a solid acid catalyst. Among these, a strongly acidic catalyst is preferable, and specific examples thereof may include sulfuric acid, hydrochloric acid, nitric acid, sulfamic acid, methanesulfonic acid, and crosslinked polystyrene sulfonic acid.

As the amount of the acid catalyst used, the total amount of the amount required to neutralize the basic catalyst contained in N-(α-hydroxyethyl)formamide and the amount required to advance the alkoxylation reaction is required. The amount of the acid catalyst used as the total amount is preferably a molar amount to be from 1.01 to 10 times the amount required to neutralize the basic catalyst and more preferably a molar amount to be from 1.1 to 5 times the amount required to neutralize the basic catalyst.

After the completion of the alkoxylation reaction, the acid catalyst is usually neutralized with an alkali compound or it is filtered and separated in a case in which the acid catalyst is in the form of solid such as an ion exchange resin. Incidentally, the neutralization treatment itself is not an essential operation, but it is preferable to conduct the neutralization treatment from the viewpoint of minimizing the decomposition of N-(α-alkoxyethyl)formamide which is a product in the purification and recovery step since N-(α-alkoxyethyl)formamide is more stable under a neutral condition.

Incidentally, as described above, the basic catalyst in the N-(α-hydroxyethyl)formamide obtained in the step (2) in the (method for producing N-(α-alkoxyethyl)formamide) reacts with sulfuric acid to form a sulfate such as sodium sulfate or potassium sulfate, for example, in the case of using sulfuric acid as the acid catalyst in the alkoxylation reaction. This sulfate hardly dissolves in the reaction mixture after the completion of the alkoxylation reaction, and the sulfate can be thus separated from the N-(α-alkoxyethyl)formamide by using a filter or the like.

It is preferable that the N-(α-alkoxyethyl)formamide obtained in the step (3) is purified by distillation to remove impurities and the like after the acid catalyst is neutralized or removed in the case of a solid acid.

The method for producing N-(α-alkoxyethyl)formamide of the invention may be a batch type in which a series of reactions (hydroxylation reaction and alkoxylation reaction) are discontinuously conducted or a continuous type in which a series of reactions are continuously conducted.

In the case of a batch type, it is preferable to measure the basic catalyst by the amount to be calculated as the amount of catalyst required for one time of reaction and to store the basic catalyst in the first tank.

### <Effect>

As described above, according to the method for producing N-(α-alkoxyethyl)formamide of the invention, impurities in the N-(α-alkoxyethyl)formamide decrease since N-(α-hydroxyethyl)formamide obtained by the method for producing N-(α-hydroxyethyl)formamide of the invention is used as a raw material in this method. The N-(α-alkoxyethyl)formamide thus obtained is a raw material for N-vinylformamide exhibiting excellent purity and quality.

### (Device for producing N-(α-hydroxyethyl)formamide)

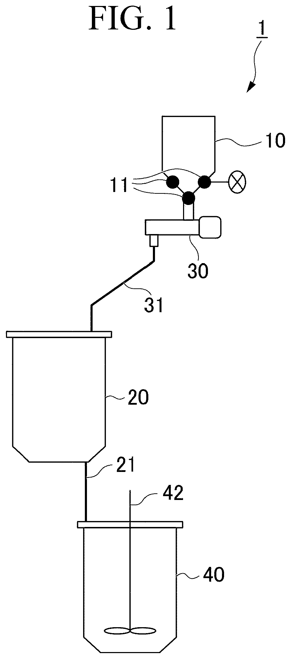

As illustrated in Fig. 1, a device 1 for producing N-(α-hydroxyethyl)formamide of the invention (hereinafter simply referred to as the "production device 1") is equipped with a catalyst storage facility for storing a basic catalyst, a mixing tank 20 provided downstream of the catalyst storage facility, a third tank 40 (hydroxylation reaction tank) provided downstream of the mixing tank 20, and a vibration imparting means 11 for vibrating at least a part of the catalyst storage facility when the basic catalyst is supplied from the catalyst storage facility to the mixing tank 20.

The catalyst supply facility includes a catalyst storage tank 10 for storing a basic catalyst, a powder feeder 30 for supplying the basic catalyst, and a catalyst supply pipe 31 for supplying the basic catalyst.

The powder feeder 30 and the catalyst supply pipe 31 are provided between the catalyst storage tank 10 and the mixing tank 20.

A catalyst-mixed liquid supply pipe 21 is provided between the mixing tank 20 and the third tank 40.

The catalyst storage tank 10 is a tank for storing a basic catalyst measured and is a tank for supplying the basic catalyst to the mixing tank 20.

The powder feeder 30 adjusts the amount of the basic catalyst supplied from the catalyst storage tank 10 to the mixing tank 20.

The catalyst supply pipe 31 connects the catalyst storage tank 10, the powder feeder 30, and the mixing tank 20 with one another and supplies the basic catalyst from the catalyst storage tank 10 to the mixing tank 20.

The mixing tank 20 is a tank for mixing formamide with the basic catalyst supplied from the catalyst storage tank 10.

The catalyst-mixed liquid supply pipe 21 connects the mixing tank 20 with the third tank 40 and supplies a mixture (reaction raw material liquid A) of a basic catalyst and formamide from the mixing tank 20 to the third tank 40.

The third tank 40 is a tank for reacting (hydroxylation reaction) the reaction raw material liquid A supplied from the mixing tank 20 with acetaldehyde and thus forming N-(α-hydroxyethyl)formamide.

It is preferable that the vibration imparting means 11 for vibrating at least a part of the catalyst supply facility is hammering. Specific examples of hammering may include an air pulse generator, an air knocker, an electric knocker, and vibration generators such as an ultrasonic vibrator, a piezoelectric vibrator, and a ball vibrator as well as a hammer, but the examples are not limited thereto. Among these, an air pulse generator, an air knocker, an electric knocker, and a vibration generator which impart vibration having a constant vibration frequency, can uniformly supply a constant amount of catalyst, and do not hinder the measuring of catalyst are preferable.

The vibration imparting means 11 imparts vibration to at least a part of the catalyst storage facility when the basic catalyst is supplied from the catalyst storage facility to the mixing tank 20. This vibration is propagated from the catalyst storage tank 10 to the powder feeder 30 and the catalyst supply pipe 31, for example, in a case in which vibration is imparted to the catalyst storage tank 10. The installation place of the vibration imparting means 11 may be the powder feeder 30 or the catalyst supply pipe 31, but it is preferably the catalyst storage tank 10, and it is most preferably the bottom portion of the catalyst storage tank 10 as illustrated in Fig. 1.

### <Effect>

As described above, the production device 1 of the invention has a vibration imparting means for vibrating at least a part of the catalyst storage facility when the basic catalyst is supplied from the catalyst storage facility to the mixing tank 20. For this reason, the amount of the basic catalyst retained at the bottom portion of the catalyst storage tank 10, in the powder feeder 30, and in the catalyst supply pipe 31 decreases and further the basic catalyst is filled in the powder feeder 30. As a result, the amount of the basic catalyst supplied can be adjusted by the powder feeder 30 while measuring the amount of the basic catalyst stored in the catalyst storage tank 10 and a regulated amount of basic catalyst can be quantitatively and sufficiently supplied to the reaction system. In addition, the solid components do not accumulate in a supply control valve 43 for the reaction slurry and the flow rate of the hydroxylation reaction slurry does not decrease.

### (Device for producing N-(α-alkoxyethyl)formamide)

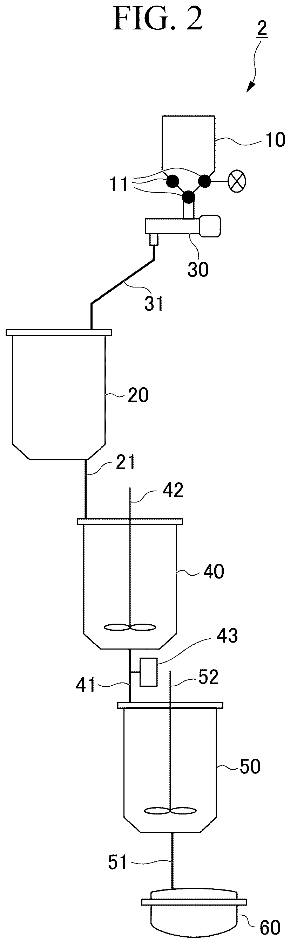

As illustrated in Fig. 2, a device 2 for producing N-(α-alkoxyethyl)formamide of the invention (hereinafter simply referred to as the "production device 2") is equipped with a catalyst supply facility for storing a basic catalyst, a mixing tank 20 provided downstream of the catalyst supply facility, a third tank 40 (hydroxylation reaction tank) provided downstream of the mixing tank 20, a fourth tank 50 (alkoxylation reaction tank) provided downstream of the third tank 40, and a vibration imparting means 11 for vibrating at least a part of the catalyst supply facility when the basic catalyst is supplied from the catalyst supply facility to the mixing tank.

Descriptions on the members common to the device 1 for producing N-(α-hydroxyethyl)formamide of the invention are omitted.

A supply pipe 41 is provided between the third tank 40 and the fourth tank 50.

A supply pipe 51 is provided between the fourth tank 50 and a filter 60.

The supply pipe 41 is provided with a supply control valve 43.

The supply pipe 41 connects the third tank 40 with the fourth tank 50 and supplies N-(α-hydroxyethyl)formamide from the third tank 40 to the fourth tank 50.

The fourth tank 50 is a tank for reacting (alkoxylation reaction) the N-(α-hydroxyethyl)formamide formed in the third tank 40 with an alcohol in the presence of an acid catalyst and thus forming N-(α-alkoxyethyl)formamide.

The supply pipe 51 connects the fourth tank 50 with the filter 60 and supplies the N-(α-alkoxyethyl)formamide from the fourth tank 50 to the filter 60.

The filter 60 separates the N-(α-alkoxyethyl)formamide and the solid phase from each other.

### <Effect>

As described above, the production device 2 of the invention has a vibration imparting means for vibrating at least a part of the catalyst storage facility when the basic catalyst is supplied from the catalyst supply facility to the mixing tank 20, and thus the amount of the basic catalyst retained at the bottom portion of the catalyst storage tank 10, in the powder feeder 30, and in the catalyst supply pipe 31 decreases and further the basic catalyst is filled in the powder feeder 30. As a result, the amount of the basic catalyst supplied can be adjusted by the powder feeder 30 while measuring the amount of the basic catalyst stored in the catalyst storage tank 10 and a regulated amount of basic catalyst can be quantitatively and sufficiently supplied to the reaction system. In addition, the solid components do not accumulate in the supply control valve 43 for the reaction slurry and the flow rate of the hydroxylation reaction slurry does not decrease. Hence, the reaction yield of N-(α-alkoxyethyl)formamide increases and impurities in the N-(α-alkoxyethyl)formamide decrease. The N-(α-alkoxyethyl)formamide thus obtained is a raw material for N-vinylformamide exhibiting excellent purity and quality.

### EXAMPLES

Hereinafter, the invention will be specifically described with reference to Examples, but the invention is not limited thereto. Incidentally, "%" represents "% by mass" unless otherwise stated.

### [Example 1]

By using the production device 2 illustrated in Fig. 2, N-(α-hydroxyethyl)formamide was produced and subsequently N-(α-alkoxyethyl)formamide was produced in the following manner.

### <Production of N-(α-hydroxyethyl)formamide>

Potassium hydrogencarbonate was stored in the catalyst storage tank 10 illustrated in Fig. 2, and 95.2 kg of formamide was charged into the mixing tank 20.

While the catalyst storage tank 10, the powder feeder 30, and the catalyst supply pipe 31 were vibrated by hammering the bottom portion of the catalyst storage tank 10 by using an air knocker (Model SK-30 manufactured by SEISHIN ENTERPRISE Co., Ltd.), potassium hydrogencarbonate stored in the catalyst storage tank 10 was supplied to the powder feeder 30 by being dropped thereonto, 1.69 kg of potassium hydrogencarbonate was supplied to the mixing tank 20 through the powder feeder 30 over 30 minutes to prepare a mixed liquid of potassium hydrogencarbonate and formamide. The total amount of potassium hydrogencarbonate retained in the first tank 10, the powder feeder 30, and the catalyst supply pipe 31 after the catalyst supply was about 15 g. This is 0.88% of the amount of the basic catalyst stored in the catalyst storage tank 10.

Separately, 384 kg of toluene for industrial use was charged into the third tank 40 which was made of glass lining and equipped with a stirrer 42 and a temperature controller (not illustrated), the third tank 40 was purged with nitrogen gas, 107 kg of acetaldehyde was then added to the toluene, and the temperature was adjusted to 20°C.

Subsequently, 20% of the mixed liquid of potassium hydrogencarbonate and formamide in the mixing tank 20 was added to the toluene solution of acetaldehyde in the third tank 40 over 15 minutes. Thereafter, the remaining amount of the mixed liquid of potassium hydrogencarbonate and formamide was further added thereto over 3 hours, the mixture was subjected to aging (hydroxylation reaction) for 1 hour to obtain a reaction slurry.

The reaction slurry obtained was transferred to the fourth tank 50 which was made of glass lining and equipped with a stirrer 52 and a temperature controller (not illustrated) and filtered to separate toluene of a solvent.

Apart of solid component (reaction mixture) filtered was sampled and analyzed by liquid chromatography under the following conditions, and as a result, the reaction mixture contained N-(α-hydroxyethyl)formamide at 64.3%, formamide at 0.7%, acetaldehyde at 1.4%, and an aldol condensate of acetaldehyde at 0.2%, and the hydroxylation reaction yield from formamide to N-(α-hydroxyethyl)formamide was 97.0%. These results are presented in Table 1.

### (Conditions for liquid chromatography analysis)

· Column: MCI-GEL-ODS 1HU (4.6 mm φ × 250 mm).
· Flow rate: 1 mL/min.
· Eluent: 0.01 M NaH₂PO₃·2H₂O aqueous solution.
· Sample injection volume: 20 µL of sample diluted with eluent by 1000 times.

### <Production of N-(α-alkoxyethyl)formamide>

To the reaction mixture containing N-(α-hydroxyethyl)formamide in the fourth tank 50, 205.2 kg of a 1.0% methanol solution of sulfuric acid was added and the reaction (methoxylation reaction) thereof was conducted at 15°C for 1 hour.

Subsequently, a 25% aqueous solution of sodium hydroxide was added to the resultant mixture until the pH reached 7 to neutralize the acid catalyst. Thereafter, potassium sulfate (inorganic salt) which was a reaction product of potassium hydrogencarbonate with sulfuric acid was separated from the mixture by using a metal filter type pressurized filter (Model AAF-5734 manufactured by Fuji Filter Manufacturing Co. Ltd) made of SUS 304 as the filter 60. The separability in solid-liquid separation at this time was favorable and the separation was completed in 30 minutes.

The filtrate obtained by this solid-liquid separation was analyzed by liquid chromatography under the above conditions, and as a result, the filtrate contained N-(α-methoxyethyl)formamide (MeO form) at 46.0%, formamide (FAM) at 0.18%, and N-(α-hydroxyethyl)formamide (OH form) at 0.95%. The percent conversion of formamide (abbreviated as the "FAM conversion" in Table 2) at this time was 99.1%, and the selectivity coefficient from formamide to N-(α-methoxyethyl)formamide (abbreviated as the "MeO form selectivity" in Table 2) was 97.7%. As a result, the total reaction yield (abbreviated as the "methoxylation reaction yield" in Table 2) of the hydroxylation reaction and methoxylation reaction from formamide to N-(α-methoxyethyl)formamide was 96.8%. These results are presented in Table 2.

### [Comparative Example 1]

N-(α-methoxyethyl)formamide was produced in the same manner as in Example 1 except that the bottom portion of the catalyst storage tank 10 was not hammered by using an air knocker. The results are presented in Table 1 and Table 2.

**[Table 1]**

| | Hammering | Amount of catalyst retained [g] | FAM [% by mass] | AAL [% by mass] | Aldol condensate [% by mass] | OH form [% by mass] | Hydroxylation reaction yield [%] |
|---|---|---|---|---|---|---|---|
| Example 1 | ○ | About 15 | 0.7 | 1.4 | 0.2 | 64.3 | 97.0 |
| Comparative Example 1 | × | About 500 | 0.9 | 1.9 | 0.2 | 63.6 | 96.0 |

**[Table 2]**

| | Hammering | Time required for solid-liquid separation [hours] | FAM [% by mass] | OH form [% by mass] | MeO form [% by mass] | FAM conversion [%] | MeO form selectivity [%] | Methoxylation reaction yield [%] |
|---|---|---|---|---|---|---|---|---|
| Example 1 | ○ | 0.5 | 0.18 | 0.95 | 46.0 | 99.1 | 97.7 | 96.8 |
| Comparative Example 1 | × | 5.0 | 0.58 | 4.95 | 43.1 | 97.4 | 88.3 | 85.9 |

Abbreviations in Table 1 and Table 2 are as follows.
- FAM: Formamide
- AAL: Acetaldehyde
- OH form: N-(α-hydroxyethyl)formamide
- MeO form: N-(α-methoxyethyl)formamide
- ○: Basic catalyst is supplied while vibrating catalyst storage tank by hammering
- ×: Basic catalyst is supplied without vibrating catalyst storage tank by hammering

As presented in Table 1, the total amount of potassium hydrogencarbonate retained in the catalyst storage tank 10, the powder feeder 30, and the catalyst supply pipe 31 after the catalyst supply in Example 1 was about 15 g and the total reaction yield of the hydroxylation reaction and methoxylation reaction from formamide to N-(α-methoxyethyl)formamide was 96.8%.

On the other hand, in the case of Comparative Example 1 in which hammering was not conducted, the total amount of potassium hydrogencarbonate retained in the catalyst storage tank 10, the powder feeder 30, and the catalyst supply pipe 31 was about 500 g and the total reaction yield of the hydroxylation reaction and methoxylation reaction from formamide to N-(α-methoxyethyl)formamide was 85.9%. In addition, the separability was poor when conducting solid-liquid separation of potassium sulfate (inorganic salt) by using the filter 60 after the alkoxylation reaction and it took 5 hours to complete the separation.

As is clear from Table 1 and Table 2, the hydroxylation reaction yield was 96.0% in Comparative Example 1 but 97.0% in Example 1, and the values of selectivity coefficient of the methoxy form and methoxylation reaction yield were remarkably favorable in Example 1 as compared with those in Comparative Example 1. It is presumed that the hydroxy form has been converted into an unknown impurity since the value of percent conversion of formamide after the methoxylation reaction in Comparative Example 1 is comparable to that in Example 1.

### INDUSTRIAL APPLICABILITY

According to the invention, it is possible to provide a method for producing N-(α-hydroxyethyl)formamide by which the amount of the basic catalyst retained at the bottom portion of the catalyst storage tank, in the powder feeder, and in the catalyst supply pipe decreases, the amount of the basic catalyst to be supplied from the catalyst storage tank to the mixing tank becomes quantitative, the flow rate of the hydroxylation reaction slurry does not decrease, and the reaction yield of hydroxylation reaction is improved, a method for producing N-(α-alkoxyethyl)formamide by which the reaction yield of alkoxylation is improved and N-(α-alkoxyethyl)formamide exhibiting excellent quality is obtained, a device for producing N-(α-hydroxyethyl)formamide, and a device for producing N-(α-alkoxyethyl)formamide.

### EXPLANATIONS OF LETTERS OR NUMERALS

- 1: DEVICE FOR PRODUCING N-(α-HYDROXYETHYL)FORMAMIDE
- 2: DEVICE FOR PRODUCING N-(α-ALKOXYETHYL)FORMAMIDE
- 10: CATALYST STORAGE TANK
- 11: VIBRATION IMPARTING MEANS
- 20: MIXING TANK
- 21: CATALYST-MIXED LIQUID SUPPLY PIPE
- 30: POWDER FEEDER
- 31: CATALYST SUPPLY PIPE
- 40: THIRD TANK
- 41: SUPPLY PIPE
- 42: STIRRER
- 43: SUPPLY CONTROL VALVE
- 50: FOURTH TANK
- 51: SUPPLY PIPE
- 52: STIRRER
- 60: FILTER

## Claims

1. A method for producing N-(α-hydroxyethyl)formamide, comprising:
a step (1) of mixing a basic catalyst stored in a catalyst supply facility with formamide in a mixing tank, wherein the catalyst supply facility includes a catalyst storage tank for storing the basic catalyst, a powder feeder for supplying the basic catalyst, and a catalyst supply pipe for supplying the basic catalyst;
a step (2) of bringing the formamide mixed with the basic catalyst into contact with acetaldehyde and obtaining N-(α-hydroxyethyl)formamide; wherein
the basic catalyst is supplied to the mixing tank while vibrating at least a part of the catalyst supply facility in the step (1) which is one of the catalyst storage tank, the powder feeder and catalyst supply pipe.

2. The method for producing N-(α-hydroxyethyl)formamide according to claim 1, wherein at least a part of the catalyst supply facility is vibrated by hammering.

3. The method for producing N-(α-hydroxyethyl)formamide according to claim 1 or 2, wherein an amount of the basic catalyst supplied from the catalyst supply facility to the mixing tank is 90% by mass or more with respect to a total mass of the basic catalyst stored in the catalyst supply facility in the step (1).

4. The method for producing N-(α-hydroxyethyl)formamide according to claim 1, wherein the basic catalyst is filled in the powder feeder and then supplied to the mixing tank in the step (1).

5. The method for producing N-(α-hydroxyethyl)formamide according to any one of claims 1 to 4, wherein the formamide and the acetaldehyde are brought into contact with each other in a state in which the basic catalyst is dissolved in the step (2).

6. A device for producing N-(α-hydroxyethyl)formamide, comprising:
a catalyst supply facility for storing a basic catalyst;
a mixing tank for mixing the basic catalyst supplied from the catalyst supply facility with formamide;
a third tank for bringing the formamide which is mixed with the basic catalyst and supplied from the mixing tank into contact with acetaldehyde and obtaining N-(α-hydroxyethyl)formamide; and
a vibration imparting means for vibrating at least a part of the catalyst supply facility,
wherein the catalyst supply facility includes a catalyst storage tank for storing the basic catalyst, a powder feeder for supplying the basic catalyst, and a catalyst supply pipe for supplying the basic catalyst, and the powder feeder and the catalyst supply pipe are provided between the catalyst storage tank and the mixing tank,
wherein the installation place of the vibration imparting means is one of the catalyst storage tank, the powder feeder and catalyst supply pipe.

7. The device for producing N-(α-hydroxyethyl)formamide according to claim 6, wherein the vibration imparting means is to vibrate the catalyst storage tank.

8. The device for producing N-(α-hydroxyethyl)formamide according to claim 6 or 7, wherein the vibration imparting means is hammering.

9. A device for producing N-(α-alkoxyethyl)formamide, comprising:
a catalyst supply facility for storing a basic catalyst;
a mixing tank for mixing the basic catalyst supplied from the catalyst supply facility with formamide;
a third tank for bringing the formamide which is mixed with the basic catalyst and supplied from the mixing tank into contact with acetaldehyde and obtaining N-(α-hydroxyethyl)formamide;
a fourth tank for bringing the N-(α-hydroxyethyl)formamide supplied from the third tank into contact with an alcohol in presence of an acid catalyst and forming N-(α-alkoxyethyl)formamide; and
a vibration imparting means for vibrating at least a part of the catalyst supply facility.

10. The device for producing N-(α-alkoxyethyl)formamide according to claim 9, wherein
the catalyst supply facility includes a catalyst storage tank for storing the basic catalyst, a powder feeder for supplying the basic catalyst, and a catalyst supply pipe for supplying the basic catalyst, and the powder feeder and the catalyst supply pipe are provided between the catalyst storage tank and the mixing tank.

11. The device for producing N-(α-alkoxyethyl)formamide according to claim 10, wherein the vibration imparting means is to vibrate the catalyst storage tank.

12. The device for producing N-(α-alkoxyethyl)formamide according to any one of claims 9 to 11, wherein the vibration imparting means is hammering.

## Patentansprüche

1. Verfahren zur Herstellung von N-(α-Hydroxyethyl)formamid, umfassend:
einen Schritt (1) zum Mischen eines basischen Katalysators, der in einer Katalysatorzufuhreinrichtung gelagert wird, mit Formamid in einem Mischbehälter, wobei die Katalysatorzufuhreinrichtung einen Katalysatorvorratsbehälter zum Lagern des basischen Katalysators, eine Pulverzuführung zum Zuführen des basischen Katalysators und eine Katalysatorzuleitung zum Zuführen des basischen Katalysators einschließt,
einen Schritt (2) zum Inkontaktbringen des mit basischen Katalysators vermischten Formamids mit Acetaldehyd und Erhalten von N-(α-Hydroxyethyl)formamid, wobei
der basische Katalysator dem Mischbehälter zugeführt wird, während mindestens ein Teil der Katalysatorzufuhreinrichtung im Schritt (1), der einer/eine von dem Katalysatorvorratsbehälter, der Pulverzuführung und der Katalysatorzuleitung ist, in Vibration versetzt wird.

2. Verfahren zur Herstellung von N-(α-Hydroxyethyl)formamid gemäß Anspruch 1, wobei mindestens ein Teil der Katalysatorzufuhreinrichtung durch Schlagen in Vibration versetzt wird.

3. Verfahren zur Herstellung von N-(α-Hydroxyethyl)formamid gemäß Anspruch 1 oder 2, wobei eine Menge des basischen Katalysators, der aus der Katalysatorzufuhreinrichtung dem Mischbehälter zugeführt wird, 90 Masse-% oder mehr, in Bezug auf eine Gesamtmasse des in der Katalysatorzufuhreinrichtung im Schritt (1) gelagerten Katalysators, beträgt.

4. Verfahren zur Herstellung von N-(α-Hydroxyethyl)formamid gemäß Anspruch 1, wobei der basische Katalysator in die Pulverzuführung gefüllt wird und dann dem Mischbehälter in Schritt (1) zugeführt wird.

5. Verfahren zur Herstellung von N-(α-Hydroxyethyl)formamid gemäß irgendeinem der Ansprüche 1 bis 4, wobei das Formamid und der Acetaldehyd in einem Zustand, in dem der basische Katalysator gelöst ist, in Schritt (2) miteinander in Kontakt gebracht werden.

6. Vorrichtung zur Herstellung von N-(α-Hydroxyethyl)formamid, umfassend:
eine Katalysatorzufuhreinrichtung zur Lagerung eines basischen Katalysators,
einen Mischbehälter zum Mischen des basischen Katalysators, der aus der Katalysatorzufuhreinrichtung zugeführt wird, mit Formamid,
einen dritten Behälter zum Inkontaktbringen des Formamids, das mit dem basischen Katalysator vermischt ist und aus dem Mischbehälter zugeführt wurde, mit Acetaldehyd und Erhalten von N-(α-Hydroxyethyl)formamid und
ein Vibration verleihendes Mittel zum Versetzen von mindestens einem Teil der Katalysatorzufuhreinrichtung in Vibration,
wobei die Katalysatorzufuhreinrichtung einen Katalysatorvorratsbehälter zum Lagern des basischen Katalysators, eine Pulverzuführung zum Zuführen des basischen Katalysators und eine Katalysatorzuleitung zum Zuführen des basischen Katalysators einschließt und die Pulverzuführung und die Katalysatorzuleitung zwischen dem Katalysatorvorratsbehälter und dem Mischbehälter angeordnet sind,
wobei der Installationsort des Vibration verleihenden Mittels einer von dem Katalysatorvorratsbehälter, der Pulverzuführung und der Katalysatorzuleitung ist.

7. Vorrichtung zur Herstellung von N-(α-Hydroxyethyl)formamid gemäß Anspruch 6, wobei das Vibration verleihende Mittel dazu dient, den Katalysatorvorratsbehälter in Vibration zu versetzen.

8. Vorrichtung zur Herstellung von N-(α-Hydroxyethyl)formamid gemäß Anspruch 6 oder 7, wobei das Vibration verleihende Mittel Schlagen ist.

9. Vorrichtung zur Herstellung von N-(α-Alkoxyethyl)formamid, umfassend
eine Katalysatorzufuhreinrichtung zur Lagerung eines basischen Katalysators,
einen Mischbehälter zum Mischen des basischen Katalysators, der aus der Katalysatorzufuhreinrichtung zugeführt wird, mit Formamid,
einen dritten Behälter zum Inkontaktbringen des Formamids, das mit dem basischen Katalysator vermischt ist und aus dem Mischbehälter zugeführt wurde, mit Acetaldehyd und Erhalten von N-(α-Hydroxyethyl)formamid, einen vierten Behälter zum Inkontaktbringen des N-(α-Hydroxyethyl)formamid, das aus dem dritten Behälter zugeführt wurde, mit einem Alkohol in Gegenwart eines Säurekatalysators und Bilden von N-(α-Alkoxyethyl)formamid und
ein Vibration verleihendes Mittel zum Versetzen von mindestens einem Teil der Katalysatorzufuhreinrichtung in Vibration,

10. Vorrichtung zur Herstellung von N-(α-Alkoxyethyl)formamid gemäß Anspruch 9, wobei
die die Katalysatorzufuhreinrichtung einen Katalysatorvorratsbehälter zum Lagern des basischen Katalysators, eine Pulverzuführung zum Zuführen des basischen Katalysators und eine Katalysatorzuleitung zum Zuführen des basischen Katalysators einschließt und die Pulverzuführung und die Katalysatorzuleitung zwischen dem Katalysatorvorratsbehälter und dem Mischbehälter angeordnet sind.

11. Vorrichtung zur Herstellung von N-(α-Alkoxyethyl)formamid gemäß Anspruch 10, wobei das Vibration verleihende Mittel dazu dient, den Katalysatorvorratsbehälter in Vibration zu versetzen.

12. Vorrichtung zur Herstellung von N-(α-Alkoxyethyl)formamid gemäß irgendeinem der Ansprüche 9 bis 11, wobei das Vibration verleihende Mittel Schlagen ist.

## Revendications

1. Procédé de production de N-(α-hydroxyéthyl)formamide, comprenant :
une étape (1) de mélange d'un catalyseur basique stocké dans une installation d'alimentation en catalyseur avec du formamide dans une cuve de mélange, dans lequel l'installation d'alimentation en catalyseur inclut une cuve de stockage de catalyseur pour stocker le catalyseur basique, un dispositif d'alimentation en poudre pour fournir le catalyseur basique, et un tuyau d'alimentation en catalyseur pour fournir le catalyseur basique ;
une étape (2) de mise du formamide mélangé avec le catalyseur basique en contact avec de l'acétaldéhyde et d'obtention de N-(α-hydroxyéthyl)formamide ; dans lequel
le catalyseur basique est fourni dans la cuve de mélange tout en faisant vibrer au moins une partie de l'installation d'alimentation en catalyseur dans l'étape (1) qui est l'un parmi la cuve de stockage de catalyseur, le dispositif d'alimentation en poudre et le tuyau d'alimentation en catalyseur.

2. Procédé de production de N-(α-hydroxyéthyl)formamide selon la revendication 1, dans lequel au moins une partie de l'installation d'alimentation en catalyseur est mise en vibration par martelage.

3. Procédé de production de N-(α-hydroxyéthyl)formamide selon la revendication 1 ou 2, dans lequel une quantité du catalyseur basique fournie par l'installation d'alimentation en catalyseur dans la cuve de mélange est de 90 % en masse ou plus par rapport à une masse totale du catalyseur basique stocké dans l'installation d'alimentation en catalyseur dans l'étape (1).

4. Procédé de production de N-(α-hydroxyéthyl)formamide selon la revendication 1, dans lequel le catalyseur basique est chargé dans le dispositif d'alimentation en poudre et ensuite introduit dans la cuve de mélange dans l'étape (1).

5. Procédé de production de N-(α-hydroxyéthyl)formamide selon l'une quelconque des revendications 1 à 4, dans lequel le formamide et l'acétaldéhyde sont mis en contact l'un avec l'autre dans un état dans lequel le catalyseur basique est dissous dans l'étape (2).

6. Dispositif de production de N-(α-hydroxyéthyl)formamide, comprenant :
une installation d'alimentation en catalyseur pour stocker un catalyseur basique ;
une cuve de mélange pour mélanger le catalyseur basique fourni par l'installation d'alimentation en catalyseur avec du formamide ;
une troisième cuve pour mettre le formamide qui est mélangé avec le catalyseur basique et fourni par la cuve de mélange en contact avec de l'acétaldéhyde et obtenir du N-(α-hydroxyéthyl)formamide ; et
un moyen de communication de vibrations pour faire vibrer au moins une partie de l'installation d'alimentation en catalyseur,
dans lequel l'installation d'alimentation en catalyseur inclut une cuve de stockage de catalyseur pour stocker le catalyseur basique, un dispositif d'alimentation en poudre pour fournir le catalyseur basique, et un tuyau d'alimentation en catalyseur pour fournir le catalyseur basique, et le dispositif d'alimentation en poudre et le tuyau d'alimentation en catalyseur sont fournis entre la cuve de stockage de catalyseur et la cuve de mélange,
dans lequel la place d'installation du moyen de communication de vibrations est l'un parmi la cuve de stockage de catalyseur, le dispositif d'alimentation en poudre et le tuyau d'alimentation en catalyseur.

7. Dispositif de production de N-(α-hydroxyéthyl)formamide selon la revendication 6, dans lequel le moyen de communication de vibrations consiste à faire vibrer la cuve de stockage de catalyseur.

8. Dispositif de production de N-(α-hydroxyéthyl)formamide selon la revendication 6 ou 7, dans lequel le moyen de communication de vibrations est un martelage.

9. Dispositif de production de N-(α-alcoxyéthyl)formamide, comprenant :
une installation d'alimentation en catalyseur pour stocker un catalyseur basique ;
une cuve de mélange pour mélanger le catalyseur basique fourni par l'installation d'alimentation en catalyseur avec du formamide ;
une troisième cuve pour mettre le formamide qui est mélangé avec le catalyseur basique et fourni par la cuve de mélange en contact avec de l'acétaldéhyde et obtenir du N-(α-hydroxyéthyl)formamide ;
une quatrième cuve pour mettre le N-(α-hydroxyéthyl)formamide fourni par la troisième cuve en contact avec un alcool en présence d'un catalyseur acide et former un N-(α-alcoxyéthyl)formamide ; et
un moyen de communication de vibrations pour faire vibrer au moins une partie de l'installation d'alimentation en catalyseur.

10. Dispositif de production de N-(α-alcoxyéthyl)formamide selon la revendication 9, dans lequel
l'installation d'alimentation en catalyseur inclut une cuve de stockage de catalyseur pour stocker le catalyseur basique, un dispositif d'alimentation en poudre pour fournir le catalyseur basique, et un tuyau d'alimentation en catalyseur pour fournir le catalyseur basique, et le dispositif d'alimentation en poudre et le tuyau d'alimentation en catalyseur sont fournis entre la cuve de stockage de catalyseur et la cuve de mélange.

11. Dispositif de production de N-(α-alcoxyéthyl)formamide selon la revendication 10, dans lequel le moyen de communication de vibrations consiste à faire vibrer la cuve de stockage de catalyseur.

12. Dispositif de production de N-(α-alcoxyéthyl)formamide selon l'une quelconque des revendications 9 à 11, dans lequel le moyen de communication de vibrations est un martelage.
